# EUROPEAN PATENT APPLICATION

(11) **EP 0 688 767 A1**
(43) Date of publication of application: **27.12.1995**
(21) Application number: 95304361.9
(22) Date of filing: 21.06.1995
(51) Int. Cl.: C07C 323/36, A61K 7/13

(54) **5-mercapto-2-nitro paraphenylenediamine compounds and hair dye compositions containing same**

(30) Priority: 24.06.1994 US 265546
(71) Applicant: Bristol-Myers Squibb Company, New York, N.Y. 10154 (US)
(72) Inventor: Lim, Mu-Ill, Trumbell Ct. 06611 (US); Pan, Yuh Guo, Stamford, Ct. 06905 (US)
(74) Representative: Cropp, John Anthony David

(57) **Abstract**

Direct dyes are disclosed, which have the structure: 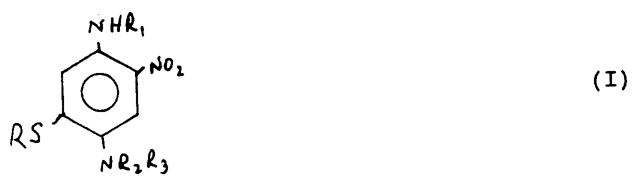
wherein R, R₁, R₂ and R₃ are the same or different and are hydrogen, C₁ to C₄ alkyl or C₁ to C₄ hydroxyalkyl having from 1 to 2 hydroxy groups, with the proviso that R is not hydrogen.

## Description

### Field of the Invention

The present invention concerns novel 5-mercapto-2-nitro paraphenylenediamines of the structure 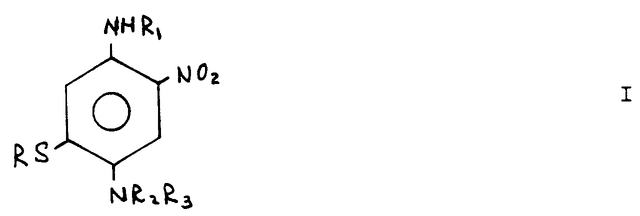
wherein R is a C₁ to C₄ alkyl or C₁ to C₄ hydroxyalkyl having from 1 to 2 hydroxy groups, with R₁, R₂ and R₃ being conventional as hereinafter defined. The present invention also concerns hair dye compositions containing compound I and methods of dyeing hair with such compositions.

### Background of the Invention

The paraphenylenediamine class of organic dye compounds is most frequently associated with the oxidative dyeing of hair, in which the paraphenylenediamine and most of its derivatives are primary intermediates that are oxidized, typically with hydrogen peroxide and in the presence of a coupler, to permanently dye hair. However, it has long been appreciated that paraphenylenediamines in which the 2-position is substituted with a strong electron withdrawing group such as the nitro group are not readily oxidizable. Accordingly they are not particularly useful as primary intermediates in oxidative hair dye products. The 2-nitro substituted paraphenylenediamines, on the other hand, are useful as direct dyes, i.e., compounds that per se have color in solution. Accordingly, the 2-nitro substituted paraphenylenediamine class of compounds is useful in formulating temporary and/or semipermanent hair color products having a wide range of colors which are available simply by changing the substitution pattern on the amino nitrogen. Thus, a brick red is produced from the parent 2-nitro-p-phenylenediamine, while a violet blue color is produced from N¹, N⁴, N⁴-trisubstituted derivatives. Numerous 2-nitro-p-phenylenediamine derivatives having electron-donating (hydroxyl, alkoxy, a short alkyl) and electron-withdrawing substituents (halogen, CF₃) in the 5-position are known.

U.S. 5,067,967 to Akram et al. discloses a 2-nitro paraphenylenediamine in which the 5-position is provided with a group selected from F or CF₃, which are electron withdrawing groups. Similarly, U.S. 4,704,474 to Konrad et al. discloses a 2-nitro paraphenylenediamine in which a halogen is provided in the 5-position. DE 35 19 304 discloses a 2-nitro paraphenylenediamine in which RO- may be substituted in any otherwise unsubstituted position on the benzene ring. DE '304 specifically discloses 2-nitro-4-[2'-hydroxyethyl)amino]-5-methoxy aniline.

U.S. 4,863,482 to Junino et al. discloses 5-substituted-2-nitro metaphenylenediamines in which the nitro group is ortho to each amine group, and in which the 5-substituent is ZR wherein Z is O, S, or NHR' and R is H, alkyl, hydroxyalkyl, polyhydroxyalkyl, etc.

U.S. 4,736,067 to Bugaut et al. discloses 5-substituted-2-nitrophenylenediamines in which a methyl group is provided in the 5-position and in which a second methyl group is provided in an otherwise unsubstituted position in the benzene ring.

DE 33 43 642 discloses bis-4,6-[(2-hydroxyethyl)mercapto]-metaphenylenediamine. U.S. 1,940,757 to Lehmann discloses 4-alkylmercaptometaphenylenediamines as an oxidative hair dye coupler.

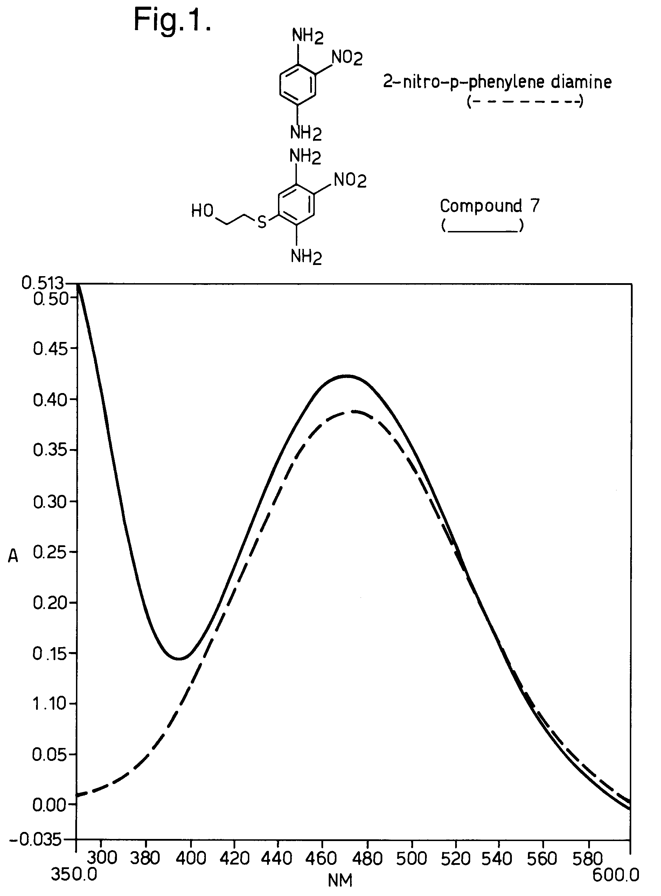

Studies concerning the effects of 4- and 5- substituents on the electronic spectra of 2-nitroaniline derivatives have shown that π-donor substituents at the 4-position cause bathochromic shifts and similar groups at the 5-position produce hypsochromic shifts (T. Yokoyama et al., J. Org. Chem. 1986, 51, 3540). Thus, 2-nitro-p-phenylenediamine has an absorption of 474.5 nm and thus provides a color in the brick-red spectrum of visible light. However, 5-methoxy-2-nitro-p-phenylenediamine disclosed in DE 35 19 304 has an absorption maximum of 460 nm and thus is much less red than 2-nitro-p-phenylenediamine, resulting from a change in the color towards the yellow spectrum of visible light.
2-Nitro-p-phenylenediamine has been used for a long time to produce a useful red shade, but it has the disadvantage that it is not stable in storage, particularly in the presence of a reducing agent such as ascorbic acid. Therefore a need exists for a stable orange-red or red direct dye that allows hair colorists to provide fashionable color.
Surprisingly, we have found that incorporation of the alkylmercapto group at the 5- position causes minimal spectral shift. 2-Nitro-p-phenylenediamine absorbs at 474.5 nm in 95% ethanol, while the compound 2,5-diamino-4-(2-hydroxyethylmercapto)-1-nitrobenzene possesses an absorption maximum of 472.6 nm in 95% ethanol (Fig. 1). More surprisingly, this novel compound dyes hair orange-red in contrast to brick-red coloration of 2-nitro-p-phenylenediamine, in spite of the fact that the spectra of these two compounds show very similar absorption maxima in the visible region.

### Summary of the Invention

It has been found that the novel compounds of the structure 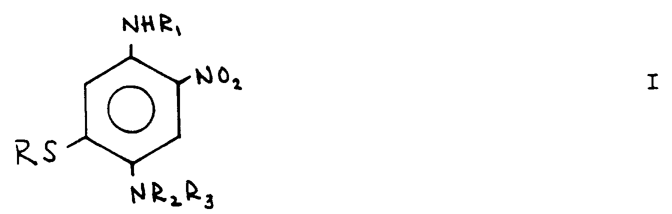
wherein R is C₁-C₄ alkyl or C₁-C₄ hydroxyalkyl having from 1 to 2 hydroxy groups are useful as semipermanent hair dyes. Advantageously, the compound I of the present invention exhibits good stability in the hair dye composition and excellent wash fastness when applied to hair.

### Description of the Drawings

Fig. 1 compares the spectra of 2-nitro-paraphenylenediamine with the spectra of 2,5-diamine-4-(2-hydroxyethylmercapto)-1-nitrobenzene of Example 1.

### Detailed Description of the Invention

The novel 5-substituted 2-nitro paraphenylenediamine compounds of the present invention have the structure 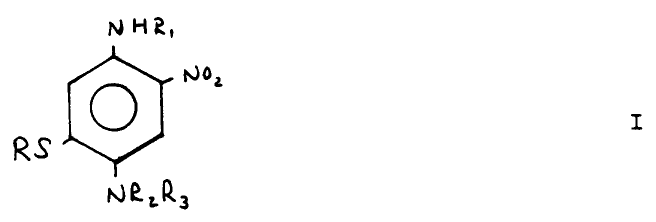
wherein R, R₁, R₂ and R₃ independently are hydrogen, C₁-C₄ alkyl, and C₁-C₄ hydroxyalkyl having 1 to 2 hydroxy groups, with the proviso that R is not hydrogen. Preferably, each of the alkyl groups of R, R₁, R₂ and R₃ have 1 or 2 carbons. The preferred compound I is 1,4-diamino-5-(2-hydroxyethylmercapto)-2-nitrobenzene, i.e., 5-(2-hydroxyethyl)mercapto-2-nitro-p-phenylenediamine.

As used herein, a "direct" dye is a compound that does not undergo chemical change in order for hair to be dyed. That is, the compound directly colors hair to which it is applied. A direct dye thus has a unique color defined by its characteristic wavelength peak. Temporary and semi-permanent hair coloring make use of such direct dyes. Temporary dyes are generally large molecules that coat the surface of the hair. The temporary dye is easily removed by one or two shampooings. Semipermanent dyes are small molecules that are rapidy absorbed into the hair shaft and similarly are slowly removed therefrom by shampooing. Three or more shampooings are typically needed to essentially completely remove the semipermanent dye from the hair shaft.

The compound I of the present invention, unlike the known 2-nitroparaphenylenediamines of the prior art, has good stability and also exhibits excellent washfastness when used to dye hair. Accordingly, the dyes of the present invention are most suitable as semipermanent direct dyes.

Moreover, the incorporation of the mercapto group in the 5-portion of the subject compound unexpectedly does not cause any significant hypsochromic shift as would be expected when an electron withdrawing group is provided at the 5- position.

Thus, 2-nitrophenylenediamine has an absorption maximum of 474.5 nm and thus provides a color in the red spectrum of visible light. The 5-methoxy-2-nitro paraphenylenediamine compound disclosed in DE 35 19 304 has an absorption maximum of 460 nm and thus is more orange than 2-nitrophenylenediamine, resulting from a change in the color towards the yellow spectrum of visible light. Advantageously, 5-(2-hydroxyethyl)mercapto-2-nitroparaphenylenediamine has an absorption maximum of 472.6 nm, i.e., the incorporation of the mercapto group at the 5-position causes essentially no hypsochromic shift in the wavelength. This is surprising because N-substitution results in a red shifting of the color (higher wavelength), while ring substitution of an electron-donating group at the 5- position is expected to cause a blue shift, as experience with the 5-methoxy compound of DE 35 19 304 demonstrates. Thus, elimination of a hypsochromic shift is beneficial in that it facilitates product development of hair dye compositions which contain a plurality of direct dyes, which in combination provide a given hair color shade. Moreover, the novel dye compounds of the present invention possess good stability and are washfast. Thus, direct hair dye compositions made with 5-mercapto substituted 2-nitroparaphenylenediamines of the present invention retain their ability to provide a given color to the hair of the consumer, and also retain that color through at least three, preferably five or more, repeated shampooings.

The hair dye composition comprises the 5-mercapto-2-nitroparaphenylenediamine direct dye of the present invention in a cosmetically acceptable vehicle. Typically, the hair dye composition contains at least one additional direct dye, the mixture of the direct dyes providing the desired hair color shade when applied to the hair of the consumer. Suitable other direct dyes include:
A. Nitroanilines, of which the following are preferred:
   2-methyl-6-nitroaniline
   N-(2-hydroxyethyl)-o-nitroaniline
   4-(2,3-dihydroxypropyl)amino-3-nitrotrifluoromethylbenzene
   4-(2-hydroxyethyl)amino-3-nitrotoluene
   4-(2-hydroxyethyl)amino-3-nitrochlorobenzene
B. Nitrophenols and phenolic ethers, of which the following are preferred:
   2-amino-3-nitrophenol
   3-amino-4-nitrophenol
   2-amino-4-nitrophenol
   2-amino-5-nitrophenol
   O,N-bis(2-hydroxyethyl)-2-amino-5-nitrophenol
   N-(2-hydroxyethyl)-2-amino-5-nitroanisole
   N-(2-hydroxyethyl)-4-amino-3-nitrophenol
   3-methylamino-4-nitrophenoxyethanol
   2-methyl-4-amino-5-nitrophenol
   3-nitro-4-(2-hydroxyethyl)aminophenoxyethanol
   1-(2-aminoethyl)amino-4-(2-hydroxyethoxy)-2-nitrobenzene
   4-bis(2-hydroxyethyl)amino-2-nitrophenol
C. Nitrophenylenediamines, and preferably:
   2-nitro-p-phenylenediamine
   4-nitro-m-phenylenediamine
   4-nitro-o-phenylenediamine
   N¹-(2-hydroxyethyl)-2-nitro-p-phenylenediamine
   N⁴-(2-hydroxyethyl)-N, N⁴-dimethyl-2-nitro-phenylenediamine
   5-chloro-N¹-(2-hydroxyethyl)-2-nitro-p-phenylenediamine
   HC Blue 2
D. Diphenylamines, such as:
   4-amino-2-nitrodiphenylamine
   4-hydroxy-2¹-nitrodephenylamine
   Acid orange 3
   2-nitro-4¹-bis(2-hydroxyethyl)aminodiphenylamine
   2-nitro-4-methoxydiphenylamine
E. Azo dyes:
   4-(p-aminophenylazo)-N-N-bis(2-hydroxyethyl)aniline
   4-(p-aminophenylazo)-3-methyl-N,N-bis(2-hydroxyethyl)aniline
   Disperse Red 17
F. Anthraquinone dyes:
   1,4,5,8-tetraaminoanthraquinone
   1,4-diaminoanthraquinone
Generally, the direct dyes are present in total amount of from 0.01 to about 5%, preferably from about 0.1 to about 3% by weight of the hair dye composition, with the 5-mercapto-2-nitroparaphenylenediamine dye of this invention being present in an amount of at least about 0.005% by weight of the composition, preferably from about 0.01 to about 3%.

Well-known conventional additives usually employed in oxidative hair coloring compositions such as thickeners, surface active agents, antioxidants and fragrances may be included in the compositions of the invention. Such compositions are preferably solutions, but they may be in the form of emulsions, suspensions, lotions or gels.

Surface active agents employed in the dyeing compositions of this invention can be anionic, nonionic, cationic or amphoteric. By way of examples of the various types of surface active agents, there can be mentioned: higher alkylbenzene sulfonates, alkylnaphthalenesulfonates, sulfonated esters or alcohols and polybasic acids, taurates, fatty alcohol sulfates, sulfates of branched chain or secondary alcohols, alkyldimethylbenzylammonium chlorides, salts of fatty acids or fatty acid mixtures, N-oxyalkylated fatty acid alkanolamides, and the like. Illustrative of specific surfactants include sodium lauryl sulfate, polyoxyethylene lauryl ester, myristyl sulfate, glyceryl monostearate, triethanolamine oleate, cetyl pyridinium chloride, lauryl sulfonate, myristyl sulfonate, lauric diethanolamide, polyoxyethylene stearate, ethoxylated oleoyl diethanolamide, polyethylene glycol amides of hydrogenated tallow, stearyldimethylbenzylammonium chloride, dodecylbenzene sodium sulfonate, triethanolamine salt of p-dodecylbenzene sulfonate, triethanolamine salt of p-dodecylbenzene sulfonate, nonylnaphthalene sodium sulfonate, dioctyl sodium sulfosuccinate, sodium N-methyl-N-oleoyl taurate, oleic acid ester of sodium isothionate, sodium dodecyl sulfate and the sodium salt of 3-diethyl-tridecanol-6-sulfate and the like. The quantity of surface active agent can vary over a wide range, such as from about 0.05% to 30% and preferably from about 0.10 to 10%.

A thickening agent may also be incorporated in the dyeing composition of this invention. The thickener may be one or several of those commonly used in hair dyeing. These are exemplified by such products as sodium alginate or gum arabic, or cellulose derivatives, such as methylcellulose, e.g., Methocel 60 HG, or the sodium salt of carboxymethylcellulose, or hydroxyethylcellulose, e.g., Cellosize QP-40 or acrylic polymers, such as polyacrylic acid sodium salt, or inorganic thickeners, such as bentonite. The quantity of thickening agent can also vary over a wide range, even as high as 20%. Ordinarily it will range from about 0.5 to 5%. The viscosity of the composition may vary from about 1 cp to about 100,000 cps. For a typical lotion formulation, composition viscosity is from about 100 cps to about 10,000 cps.

It may also be useful to incorporate an antioxidant in the present dye compositions. A variety of antioxidants known in the prior art would be useful for this purpose. Among these mention may be made of the inorganic sulfites, e.g., sodium sulfite, thioglycollic acid and other mercaptans, butylated hydroxytoluene, sodium dithionite, various forms of ascorbic acid and its derivatives, e.g., sodium ascorbate, erythorbic acid, ascorbyl palmitate, ascorbyl laurate, etc. The quantities of antioxidant employed can vary appreciably. However, the concentration will, in general, be up to about 1%, typically 0.001 to 1%.

The compounds of formula I may be prepared generally according to the reaction path illustrated by Scheme 1 of Example I in which aromatic nucleophilic substitution of 3,4-difluoronitrobenzene provides 2-fluoro-4-nitroaniline, which may be hydrogenated to yield 1,4-diamino-3-fluorobenzene which, upon acetylation, results in the formation of 2,5-diacetamido-1-fluorobenzene. Nitration of 2,5-diacetamido-1-fluorobenzene yields compound 2,5-diacetamido-4-nitro-1-fluorobenzene. Aromatic nucleophilic substitution of 2,5-diacetamido-4-nitro-1-fluorobenzene with 2-mercaptoethanol provides 2,5-diacetamido-4-(2-hydroxyethylmercapto)-1-nitrobenzene, which yields 2,5-diamino-4-(2-hydroxy ethylmercapto)-1-nitrobenzene upon hydrolysis.

The aqueous hair dyeing compositions of this invention can be prepared by conventional methods used in the hair dyeing art. Thus, they can be prepared by dissolving or suspending the components in the selected media with adequate mixing. Preparation may take place at ambient temperatures, i.e., 20° to 35°C, but solubility and rate of preparation can be enhanced utilizing elevated temperatures, for example, 40° to 100°C.

The examples which follow are illustrative of this invention.

The tristimulus values in the examples are standard Hunter chromicity values obtained by procedures well known to those skilled in the art. The values recorded manifest the ability of the compositions of the invention to be usefully employed in hair coloring processes.

In the Hunter Tristimulus system, L is a measure of lightness and darkness, that is, the depth of the color of the hair tress. The lower the value of L, the darker the color. A decrease in the value of L indicates a darkening of the hair tress. In the case of bleached and blended gray hair, a lowering of L shows deposition of hair dye on the tress.

The a value is a measure of the greenness or redness of the hair's color. As the a value increases, the hair has a more prominent red tonality. A lowering in the a value results in greener shades. The b value is a measure of the yellow and blue color. Higher b values indicate a more yellow hue in the hair. In the examples all percentages are by weight unless otherwise indicated.

### Example 1

1,4-Diamino-5-(2-hydroxyethylmercapto)-2-nitrobenzene was made by the following synthesis sequence:

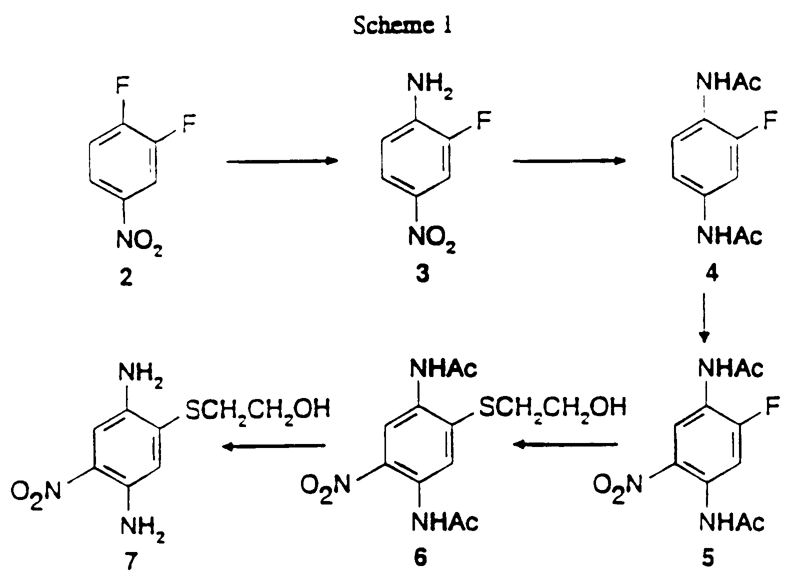

### Preparation of 2-fluoro-4-nitroaniline 3

A mixture of 3,4-difluoronitrobenzene 2 (20 g) and 30% ammonium hydroxide was placed in an autoclave at 80°C for 10 hr and cooled to room temperature. The reaction mixture was poured onto crushed ice-water (500 g). The resulting yellow precipitate was collected, washed with water and air-dried to give the product 3 as yellow powder (15.5 g, 99%); mp 135-136°C; MS m/z 156 (M⁺).

### Preparation of 2,5-diacetamiodo-1-fluorobenzene 4

A mixture of 2-fluoro-4-nitroaniline 3 (10 g) and 10% Pd-C (1 g) in ethyl acetate (200 ml) was hydrogenated at 60 psi for 1.5 hr at room temperature and filtered over a layer of Celite and washed with ethyl acetate. The combined filtrate was treated with acetic anhydride (20 ml) and the mixture was stored at room temperature for 16 hr. Evaporation of the solvent followed by trituration with hexane gave 4 as white powder (12.0 g, 89%); mp 266-268°C; ¹H NMR (DMSO-d₆, 300 MHz) δ2.01, 2.10 (2s, 3H each), 7.14 (d, 1H, J = 9 Hz), 7.65 (m, 2H), 9.59, 10.07 (2s, 1H each); MS m/z 210 (M⁺).

### Preparation of 2,5-diacetamido-4-nitro-1-fluorobenzene 5

To a stirred solution of 2,5-diacetamido-1-fluorobenzene 4 (9.5 g) in trifluoroacetic acid (50 ml) in an ice-bath was added dropwise fuming nitric acid (3 ml) over a period of 15 min. The reaction mixture was stirred for another 15 min. and poured onto crushed ice (150 g). The resulting yellow powder was collected, washed with water three times and air-dried to give the product 5 (10.1 g, 87.6%); mp 219-220°C; ¹H NMR (DMSO-d₆, 300 MHz) δ2.07, 2.10 (2s, 3H each), 7.70 (d, 1H, J = 12 Hz), 8.66 (d, 1H, J = 8 Hz), 10.07, 10.24 (2s, 1H each); MS m/z 255 (M⁺).

### Preparation of 2,5-diacetamido-4-(2-hydroxyethylmercapto)-1-nitrobenzene 6

A mixture of 2,5-diacetamido-4-nitro-1-fluorobenzene 5 (5.1 g), 2-mercaptoethanol (1.88 g) and triethylamine (3.04 g) in DMF (20 ml) was stirred for 1.5 hr at room temperature. The mixture was poured onto crushed ice. The resulting yellow precipitate was collected, washed with water three times, and air-dried to give the product. Recrystallization of the product from methanol gave the compound 6 as yellow solid (5.2 g, 83%); mp 183-184°C; ¹H NMR (DMSO-d₆, 300 MHz) δ2.06 (2, 6H), 3.06 (5, 2H, J = 6 Hz), 3.64 (m, 2H), 5.09 (5, 1H, J = 5 Hz), 7.63 (s, 1H), 7.98 (s, 1H), 9.58, 10.24 (2s, 1H each); MS m/z 313 (M⁺).

### Preparation of 2,5-diamino-4-(2-hydroxyethylmercapto)-1-nitrobenzene 7

A suspension of 2,5-diacetamido-4-(2-hydroxyethylmercapto)-1-nitrobenzene 6 (2 g) in 6N HCl (20 ml) was stirred for 1.5 hr at 90°C and poured onto crushed ice. The mixture was neutralized with 50% sodium hydroxide solution in an ice-bath. The resulting reddish powder was collected, washed with water and air-dried to give the product 7 as red powder (1.4 g, 96%); mp 160°C (140°C collapsed); ¹H NMR (DMSO-d₆, 300 MHz) δ3.02 (t, 2H, J = 6 Hz), 3.62 (t, 2H, J = 6 Hz), 4.92 (bs, 3H, exch. with D₂0), 6.84 (s, 1H), 6.92 (bs, 2H, exch. with D₂0), 7.26 (s, 1H); MS m/z 229 (M⁺).
A comparison of the spectra of compound 7 with the spectra of 2-nitrophenylenediamine is provided in Fig. 1.

### Example 2

The absorption maxima were measured for various compounds in solution by spectrophotometry.

| | λ max (nm) |
|---|---|
| 2-nitro paraphenylenediamine | 474.5 |
| 5-methoxy-2-nitroparaphenylenediamine | 460 |
| Compound of Example 1 | 472.6 |

In spite of the presence of the electron donating 2-hydroxyethylmercapto group at the C-5 position for the compound of Example 1, the λ max is similar to that of 2-nitrophenylenediamine. This is unexpected because the 2-hydroxyethylmercapto group was expected to reduce λ max in the same manner as the 5-methoxy group in 5-methoxy-2-nitroparaphenylenediamine. The shift in the λ max from 474.5 for 2-nitro-p-phenylenediamine to 460 for 5-methoxy-2-nitro-p-phenylenediamine results in a color change from red to orange.
The following composition A was used to color hair:

| | |
|---|---|
| Compound 7 | 1.00 |
| Erythrobic acid | 0.025 |
| Citric acid | 0.49 |
| Aminomethyl propanol | 1.50 |
| Lauramide DEA | 2.94 |
| Hydroxyethylcellulose | 1.35 |
| i-Propanol | 0.735 |
| Deionized water | 91.96 |

The Composition A was used to dye swatches of blended gray hair in accordance with the following procedure:
The hair was soaked in the dye solution at room temperature for 30 minutes and then rinsed with water and dried. The Composition A was found to dye hair orange-red.

For comparison purposes, the following composition (Composition B) containing 2-nitro-p-phenylenediamine was formulated.

| | |
|---|---|
| 2-Nitro-p-phenylenediamine | 0.125 g |
| Erythrobic acid | 0.025 |
| Citric acid | 0.49 |
| Aminomethyl propanol | 1.50 |
| Lauramide DEA | 2.94 |
| Hydroxyethylcellulose | 1.35 |
| i-Propanol | 0.735 |
| Deionized water | 92.835 |

The composition has a pH of 9.5. Bleached and blended gray hair were dyed as described above for Composition A to give orange-red coloration.

| Composition | Hair Type | Hunter Tristimulus Values | | |
|---|---|---|---|---|
| | | L | a | b |
| Composition A | G | 26.95 | 8.03 | 8.02 |
| Composition A | B | 35.64 | 27.13 | 17.01 |
| Composition B | G | 21.16 | 9.97 | 6.48 |
| Composition B | B | 34.46 | 26.55 | 15.38 |
| G: Blended gray hair B: Bleached hair | | | | |

The dyed hair (bleached hair) was subjected to a series of five shampoos and water rinses to determine washfastness. Washfastness results were excellent.

| Composition | Hair Type | Hunter Tristimulus Values | | |
|---|---|---|---|---|
| | | L | a | b |
| Composition A | B | 46.20 | 19.14 | 17.45 |
| Composition B | B | 46.64 | 17.50 | 16.04 |
| B: Bleached hair | | | | |

## Claims

1. A direct dye having the structure: 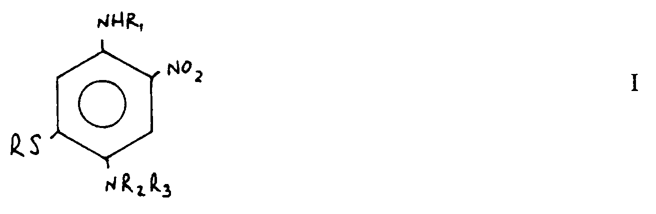 wherein R is C₁ to C₄ alkyl or C₁ to C₄ hydroxyalkyl having from 1 to 2 hydroxy groups, and R₁, R₂ and R₃ are the same or different and are hydrogen, C₁ to C₄ alkyl or C₁ to C₄ hydroxyalkyl having from 1 to 2 hydroxy groups.

2. The direct dye of Claim 1 wherein R is methyl or ethyl or a C₁ to C₃ monohydroxyalkyl, and R₁, R₂ and R₃ are hydrogen or C₁ to C₃ hydroxyalkyl having from 1 to 2 hydroxy groups, at least one of R₁, R₂ or R₃ being hydrogen.

3. The direct dye of Claim 2 wherein R is 2-hydroxyethyl and R₁, R₂ and R₃ are hydrogen.

4. A hair dye composition comprising, in a cosmetically acceptable vehicle, from 0.005 to 5% by weight of a direct dye as claimed in Claim 1, Claim 2 or Claim 3.

5. The hair dye composition of Claim 4 further comprising at least one additional direct dye, the total concentration of direct dye in the composition being from about 0.01 to about 5% by weight.
